# DEMANDE DE BREVET EUROPEEN

(11) **EP 0 953 340 A1**
(43) Date de publication de la demande: **03.11.1999**
(21) Numéro de dépôt: 99400273.1
(22) Date de dépôt: 05.02.1999
(51) Int. Cl.: A61K 7/48

(54) **Utilisation d'amidon comme actif destiné au traitement et/ou à la prévention de l'apparition de signes du vieillissement dans une composition cosmétique ou dermatologique**

(30) Priorité: 13.03.1998 FR 9803151
(71) Demandeur: L'OREAL, 75008 Paris (FR)
(72) Inventeur: Chevalier, Véronique, 94440 Villecresnes (FR); Bordeaux, Dominique, 91310 Longpont Sur Orge (FR)
(74) Mandataire: Renard, Emmanuelle

(57) **Abrégé**

L'invention se rapporte à l'utilisation d'amidon dans une composition topique comme actif pour traiter et/ou pour prévenir l'apparition des rides, des ridules, du relâchement cutané et/ou sous cutané et/ou pour raviver l'éclat de la peau. Elle se rapporte aussi à un procédé de traitement cosmétique des rides, ridules, du relâchement de la peau et de son éclat.

## Description

L' invention se rapporte à l'utilisation d'amidon, dans une composition cosmétique ou pour la préparation d'une composition dermatologique à application topique, comme actif destiné à la prévention et/ou au traitement de certains signes du vieillissement endogène et/ou exogène.

Le vieillissement cutané résulte des effets sur la peau de facteurs intrinsèques et extrinsèques. Cliniquement, les signes du vieillissement se traduisent par l'apparition de rides et ridules, par un relâchement des tissus cutanés et sous-cutanés, par une perte de l'élasticité cutanée, par une atonie de la texture de la peau, et par le jaunissement de la peau qui devient plus terne et sans éclat.

Certains de ces signes sont plus particulièrement liés au vieillissement intrinsèque ou physiologique, c'est-à-dire au vieillissement lié à l'âge, alors que d'autres sont plus spécifiques du vieillissement extrinsèque, c'est-à-dire du vieillissement provoqué d'une manière générale par l'environnement; il s'agit plus particulièrement du photo-vieillissement dû à l'exposition au soleil, à la lumière ou à tout autre rayonnement.

Les changements de la peau résultant du vieillissement intrinsèque ou physiologique sont la conséquence d'une sénescence génétiquement programmée où interviennent des facteurs endogènes. Ce vieillissement intrinsèque provoque notamment un ralentissement du renouvellement des cellules de la peau. Histologiquement la peau est globalement amincie, tant au niveau épidermique que dermique. La densité des macromolécules fibreuses du derme (élastine et collagène) est diminuée. Au contraire, le vieillissement extrinsèque entraîne des changements histopathologiques tels qu'une excessive accumulation de matière élastique dans le derme supérieur et une dégénérescence des fibres de collagène.

L'invention s'intéresse essentiellement aux rides, ridules, au relâchement des tissus cutanés et sous-cutanés et à l'éclat de la peau. Le relâchement des tissus cutanés et sous-cutanés se traduit par une texture de peau atone, un relâchement du microrelief cutané, une fermeté cutanée diminuée, et une peau globalement flasque.

On connaît de nombreux actifs qui prétendent traiter les rides et les ridules de la peau ou raffermir les tissus cutanés, mais ces actifs ne traitent qu'incomplètement et temporairement ces désordres morphologiques. Aussi, il subsiste le besoin d'actifs destinés à une application topique permettant de traiter plus efficacement les rides et les ridules ainsi que de raffermir les tissus cutanés, L'invention permet, de plus, de donner un éclat à la peau âgée, comparable à celui d'une peau plus jeune.

L'invention a donc pour objet l'utilisation d'amidon dans une composition cosmétique ou pour la préparation d'une composition dermatologique comme actif destiné à traiter et/ou prévenir l'apparition des rides, des ridules, du relâchement cutané et/ou sous-cutané et/ou pour raviver l'éclat de la peau.

Plus particulièrement, l'invention se rapporte à l'utilisation d'amidon dans une composition topique ou pour la préparation d'une composition dermatologique comme actif destiné à traiter et/ou prévenir l'apparition du relâchement et/ou l'avachissement ou l'effondrement du microrelief cutané et/ou sous-cutané, de la flaccidité cutanée et/ou sous-cutanée, de la perte de l'élasticité cutanée et/ou sous-cutanée et/ou pour raffermir la peau et/ou tonifier la texture de la peau.

L'invention a aussi pour objet un procédé de traitement cosmétique des rides et/ou des ridules et/ou du relâchement cutané et/ou sous-cutané en vue de raffermir la peau et/ou pour raviver l'éclat de la peau, comprenant l'application sur la peau d'amidon dans une composition cosmétique.

L'invention a aussi pour objet un procédé cosmétique de prévention de l'apparition des rides et/ou des ridules et/ou du relâchement cutané et/ou sous-cutané en vue de raffermir la peau et/ou pour raviver l'éclat de la peau, comprenant l'application sur la peau d'amidon dans une composition cosmétique.

On connaît par le document EP-281395 des compositions cosmétiques susceptibles d'applanir les rides, ces compositions comprenant un copolymère amidon-acrylate. Toutefois, le copolymère amidon-acrylate est présent comme actif destiné à favoriser l'hydratation de la peau, et non comme actif susceptible de prévenir ou de traiter l'apparition des signes du vieillissement cutané.

De nombreux documents décrivent des compositions cosmétiques destinées au traitement ou à la prévention de l'apparition des signes du vieillissement cutané tels que les rides et les ridules, le relâchement de la peau, ces compositions comprenant de l'amidon ou un polymère dérivé d'amidon ou un polymère apparenté à l'amidon. C'est le cas des documents suivants: WO 90/04383, EP 761203, EP 738510, WO 9421299, WO 93/10756, EP 552624, WO 93/10755, US 5017367, FR 2659551. Toutefois dans ces documents l'amidon est toujours employé comme support de compositions cosmétiques, son utilisation comme actif destiné au traitement ou à la prévention de l'apparition des signes du vieillissement cutané n'est jamais envisagée.

Le document WO 96/29080 décrit des polymères de toutes structures, parmi lesquels de l'amidon, ces polymères étant greffés par des résidus alpha hydroxy acides, destinés au traitement des signes du vieillissement cutané. Toutefois, dans ce document, c'est l'alpha hydroxy acide qui est considéré comme actif anti-rides, le polymère servant de support destiné à faciliter la formulation et le transport de l'alpha hydroxy acide.

La composition contient un milieu cosmétiquement ou dermatologiquement acceptable, c'est-à-dire compatible avec les tissus cutanés. Ainsi, la composition peut être appliquée sur tout le corps humain.

L'amidon est un produit naturel bien connu de l'homme du métier. Il consiste en un polymère ou un mélange de polymères, linéaires ou branchés, constitués d'unités d'α-D-glucopyranosyle. L'amidon est décrit en particulier dans "KIRK-OTHMER ENCYCLOPEDIA OF CHEMICAL TECHNOLOGY, 3^{ème} édition, volume 21, p.492-507, Wiley lnterscience, 1983".

L'amidon employé selon la présente invention peut être de toute origine : riz, maïs, pomme de terre, manioc, pois, froment, avoine etc...

Il peut être naturel ou éventuellement modifié par un traitement du type réticulation, acétylation, oxydation.

Selon l'invention, l'amidon est utilisé en une quantité allant de 0,1 % à 20 % du poids total de la composition, de préférence de 0,5 % à 15 % et mieux de 1 % à 10 %.

Les compositions utilisables dans l'invention peuvent se présenter sous toutes les formes galéniques classiquement utilisées pour une application topique et notamment sous forme de solutions aqueuses ou hydroalcooliques, de dispersions du type lotion ou sérum, de gels aqueux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique ou dermatologique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

De façon connue, la composition cosmétique ou dermatologique de l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides autres que l'amidon tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène.

Comme actifs lipophiles ou hydrophiles utilisables dans l'invention en vue de parfaire le traitement des rides, ridules, la lutte contre le relâchement cutané et/ou sous-cutané et/ou l'éclat de la peau, on peut citer par exemple les rétinoïdes (rétinol et ses esters, rétinal, acide rétinoïque et ses dérivés, rétinoïdes, notamment ceux décrits dans les documents FR-A-2 570 377, EP-A-199636, EP-A-325540, EP-A-402072), les α-hydroxy-acides (glycolique, lactique, malique, citrique, tartrique, mandélique), les β-hydroxy-acides (acide salicylique et ses dérivés notamment alcoylés), les α-céto-acides, les β-céto-acides, les peroxydes comme le peroxyde de benzoyle, les vitamines notamment E, F, les actifs anti-radicaux comme la superoxyde-dismutase, le sélénium, le zinc, les béta-carotènes, les polymères tenseurs, qu'ils soient d'origine naturelle ou synthétique

La composition peut en outre contenir des hormones, naturelles ou synthétiques, oestrogéniques, progestatives ou androgéniques comme la progestérone, la testostérone, l'oestradiol anhydre, le broparestrol, l'oestrone, l'acétate de prégnénolone, la prégnénolone, la 17 béta-hydroxy- progestérone, le propionate de testostérone, l'androstènedione et les androstanediols.

Les exemples suivants illustrent l'invention. Dans ces exemples, les proportions indiquées sont des pourcentages en masse, les noms des constituants sont des noms INCI.

### Exemple 1: Crème anti-rides (émulsion eau-dans-huile)

| Phase A : | |
|---|---|
| Hydrogenated polyisobutene | 5,5 % |
| Isostearyl neopentanoate | 3,5 % |
| PEG-20 stearate | 1 % |
| Glyceryl stearate et PEG-100 stearate | 2 % |
| Cetyl alcohol | 0,5 % |
| Stearyl alcohol | 0,5 % |
| Stearic acid | 1 % |

| Phase A': | |
|---|---|
| Cyclomethicone | 11 % |

| Phase C : | |
|---|---|
| Polyacrylamide et C13-C14 isoparaffin et laureth-7 | 1 % |

| Phase D : | |
|---|---|
| Amidon de riz (commercialisé par la société Lambert-Rivière) | 7 % |
| Eau | 25 % |

| Phase B: | |
|---|---|
| Conservateurs | qs |
| Triéthanolamine | 0,03 % |
| Eau | qsp 100 % |

### Mode opératoire :

On chauffe la phase A sous agitation jusqu'à homogénéité. Après refroidissement on ajoute la phase A'. On chauffe la phase B sous agitation, puis on verse B dans A toujours sous agitation. Après refroidissement à 50°C on incorpore à l'émulsion la phase C. On prépare un empois d'amidon en cuisant la phase D pendant 1 heure à 80°C. Cet empois est finalement incorporé au reste de la préparation à 40°C.

### Exemple 2 : Sérum anti-vieillissement

| | |
|---|---|
| Polyacrylamide et C13-C14 isoparaffin et laureth-7 | 1 % |
| Xanthan gum | 0,2 % |
| PVM/MA Decadiene Crosspolymer | 0,2 % |
| Triethanolamine | 0,2 % |
| Amidon de maïs réticulé (C TEX 06205 commercialisé par la société Cérestar) | 3,5 % |
| Conservateur | qs |
| Eau | qsp 100 % |

Mode opératoire : On prépare un empois d'amidon en cuisant pendant 1 heure l'amidon dans 40% d'eau (en poids par rapport au poids total de la préparation). On disperse la gomme de xanthane et le polymère (PVM/MA Decadiene Crosspolymer) dans le reste de l'eau à chaud sous agitation avec les conservateurs et la triéthanolamine. On refroidit à 40°C et on incorpore l'empois d'amidon et le polyacrylamide, toujours sous agitation.

## Revendications

1. Utilisation d'amidon dans une composition cosmétique ou pour la préparation d'une composition dermatologique à application topique, comme actif destiné à la prévention et/ou au traitement des signes du vieillissement endogène et/ou exogène.

2. Utilisation d'amidon dans une composition cosmétique ou pour la préparation d'une composition dermatologique à application topique, comme actif destiné à traiter et/ou prévenir l'apparition des rides, des ridules, du relâchement cutané et/ou sous-cutané.

3. Utilisation d'amidon dans une composition cosmétique ou pour la préparation d'une composition dermatologique à application topique, comme actif destiné à traiter et/ou prévenir l'apparition du relâchement et/ou l'avachissement ou l'effondrement du microrelief cutané et/ou sous-cutané, de la flaccidité cutanée et/ou sous-cutanée, de la perte de l'élasticité cutanée et/ou sous-cutanée.

4. Utilisation d'amidon dans une composition cosmétique ou pour la préparation d'une composition dermatologique à application topique, comme actif destiné à raffermir la peau et/ou tonifier la texture de la peau et/ou raviver l'éclat de la peau.

5. Utilisation selon l'une quelconque des revendications 1 à 4 précédentes caractérisée en ce que la composition contient de 0,1 % à 20 % d'amidon, de préférence de 0,5 % à 15 % et mieux de 1 % à 10 %, en poids par rapport au poids total de la composition.

6. Procédé de traitement cosmétique des rides et/ou des ridules et/ou du relâchement cutané et/ou sous-cutané en vue de raffermir la peau et/ou pour raviver l'éclat de la peau, comprenant l'application sur la peau d'amidon dans une composition cosmétique.
